# EUROPEAN PATENT SPECIFICATION

(11) **EP 1 790 216 B1**
(45) Date of publication and mention of the grant of the patent: **21.04.2010**
(21) Application number: 05765137.4
(22) Date of filing: 27.06.2005
(51) Int. Cl.: A01H 5/00, C12N 15/12, G01N 33/74

(54) **METHOD OF DETECTING ESTROGEN-LIKE SUBSTANCE BY USING PLANT**
VERFAHREN ZUM NACHWEISEN EINER ÖSTROGENARTIGEN SUBSTANZ MIT HILFE EINER PFLANZE
PROCÉDÉ DE DÉTECTION D'UNE SUBSTANCE RESSEMBLANT À UN ESTROGÈNE EN UTILISANT UNE PLANTE

(30) Priority: 02.07.2004 JP 2004196404
(43) Date of publication of application: 30.05.2007
(73) Proprietor: National University Corporation Hokkaido University, Sapporo-shi, Hokkaido 060-0808 (JP)
(72) Inventor: YAMAZAKI, Kenichi National University Corporation, Sapporo-shi, Hokkaido 0600810 (JP); TUDA, Kenichi c/o Yamazaki Laboratory Graduate School envir. Earth Science, Kita-ku Sapporo-shi, Hokkaido 0600810 (JP); TOUJYO, Takuto National University Corporation, Sapporo-shi, Hokkaido 0600810 (JP); WADA, Tomoko National University Corporation, Sapporo-shi, Hokkaido 0600810 (JP); YAMASHITA, Kazunori, Hokkaido 0040021 (JP)
(74) Representative: Wilson Gunn
(86) International application number: PCT/JP2005/011706
(87) International publication number: WO 2006/003854

(56) References cited:
- EP-A- 0 813 604
- JP-A- 2002 247 986
- US-A1- 2004 043 394
- TOJO ET AL: "A simple and extremely sensitive system for detecting estrogenic activity using transgenic Arabidopsis thaliana" ECOTOXICOLOGY AND ENVIRONMENTAL SAFETY, ACADEMIC PRESS, SAN DIEGO, CA, US, vol. 64, no. 2, 1 June 2006 (2006-06-01), pages 106-114, XP005454932 ISSN: 0147-6513
- ZUO J. ET AL.: 'Technical advance: An estrogen receptor-based transactivator XVE mediates highly inducible gene expression in transgenic plants.' PLANT J. vol. 24, no. 2, 2000, pages 265 - 273, XP000990719
- NISHIKAWA J. ET AL.: 'New screening methods for chemicals with hormonal activities using interaction of nuclear hormone receptor with coactivator.' TOXICOL. APPL. PHARMACOL. vol. 154, no. 1, 1999, pages 76 - 83, XP001097129

### SEQUENCE LISTING

<110> NATIONAL UNIVERSITY CORPORATION HOKKAIDO UNIVERSITY
<120> METHOD FOR DETECTING ESTROGEN-LIKE CHEMICALS BY PLANT
<130> FS05-434PCT
<160> 26
<170> PatentIn version 3.1
<210> 1
   <211> 20
   <212> DNA
   <213> Artificial sequence
<220>
   <223> primer
<400> 1
   ggaagcttgc atgctgcagg 20
<210> 2
   <211> 26
   <212> DNA
   <213> Artificial sequence
<220>
   <223> primer
<400> 2
   gggctagcga cctttctctt cttctt 26
<210> 3
   <211> 26
   <212> DNA
   <213> Artificial sequence
<220>
   <223> primer
<400> 3
   gggctagcat gaaagcgtta acggcc 26
<210> 4
   <211> 27
   <212> DNA
   <213> Artificial sequence
<220>
   <223> primer
<400> 4
   gggccggcct ggttcaccgg cagccac 27
<210> 5
   <211> 202
   <212> PRT
   <213> Escherichia coli
<400> 5
   Met Lys Ala Leu Thr Ala Arg Gln Gln Glu Val Phe Asp Leu Ile Arg
   1 5 10 15
   Asp His Ile Ser Gln Thr Gly Met Pro Pro Thr Arg Ala Glu Ile Ala
   20 25 30
   Gln Arg Leu Gly Phe Arg Ser Pro Asn Ala Ala Glu Glu His Leu Lys
   35 40 45
   Ala Leu Ala Arg Lys Gly Val Ile Glu Ile Val Ser Gly Ala Ser Arg
   50 55 60
   Gly Ile Arg Leu Leu Gln Glu Glu Glu Glu Gly Leu Pro Leu Val Gly
   65 70 75 80
   Arg Val Ala Ala Gly Glu Pro Leu Leu Ala Gln Gln His Ile Glu Gly
   85 90 95
   His Tyr Gln Val Asp Pro Ser Leu Phe Lys Pro Asn Ala Asp Phe Leu
   100 105 110
   Leu Arg Val Ser Gly Met Ser Met Lys Asp Ile Gly Ile Met Asp Gly
   115 120 125
   Asp Leu Leu Ala Val His Lys Thr Gln Asp Val Arg Asn Gly Gln Val
   130 135 140
   Val Val Ala Arg Ile Asp Asp Glu Val Thr Val Lys Arg Leu Lys Lys
   145 150 155 160
   Gln Gly Asn Lys Val Glu Leu Leu Pro Glu Asn Ser Glu Phe Lys Pro
   165 170 175
   Ile Val Val Asp Leu Arg Gln Gln Ser Phe Thr Ile Glu Gly Leu Ala
   180 185 190
   Val Gly Val Ile Arg Asn Gly Asp Trp Leu
   195 200
<210> 6
   <211> 606
   <212> DNA
   <213> Escherichia coli
<400> 6
   atgaaagcgt taacggccag gcaacaagag gtgtttgatc tcatccgtga tcacatcagc 60
   cagacaggta tgccgccgac gcgtgcggaa atcgcgcagc gtttggggtt ccgttcccca 120
   aacgcggctg aagaacatct gaaggcgctg gcacgcaaag gcgttattga aattgtttcc 180
   ggcgcatcac gcgggattcg tctgttgcag gaagaggaag aagggttgcc gctggtaggt 240
   cgtgtggctg ccggtgaacc acttctggcg caacagcata ttgaaggtca ttatcaggtc 300
   gatccttcct tattcaagcc gaatgctgat ttcctgctgc gcgtcagcgg gatgtcgatg 360
   aaagatatcg gcattatgga tggtgacttg ctggcagtgc ataaaactca ggatgtacgt 420
   aacggtcagg tcgttgtcgc acgtattgat gacgaagtta ccgttaagcg cctgaaaaaa 480
   cagggcaata aagtcgaact gttgccagaa aatagcgagt ttaaaccaat tgtcgttgac 540
   cttcgtcagc agagcttcac cattgaaggg ctggcggttg gggttattcg caacggcgac 600
   tggctg 606
<210> 7
   <211> 29
   <212> DNA
   <213> Artificial sequence
<220>
   <223> primer
<400> 7
   ggggccggcc gtctgctgga gacatgaga 29
<210> 8
   <211> 27
   <212> DNA
   <213> Artificial sequence
<220>
   <223> primer
<400> 8
   gggctcagca tccaacaagg cactgac 27
<210> 9
   <211> 60
   <212> DNA
   <213> Artificial sequence
<220>
   <223> primer
<400> 9
   gggctgagcc ccccatactc tattccgagt atgatcctac cagacccttc agtgaggctt 60
<210> 10
   <211> 28
   <212> DNA
   <213> Artificial sequence
<220>
   <223> primer
<400> 10 28
   gggcggccgc tcagactgtg gcagggaa 28
<210> 11
   <211> 28
   <212> DNA
   <213> Artificial sequence
<220>
   <223> primer
<400> 11 28
   gggcggccgc gaatttcccc gatcgttc 28
<210> 12
   <211> 32
   <212> DNA
   <213> Artificial sequence
<220>
   <223> primer
<400> 12
   gggaattcac tagtccgatc tagtaacata ga 32
<210> 13
   <211> 20
   <212> DNA
   <213> Artificial sequence
<220>
   <223> primer
<400> 13
   ggaagcttgc atgctgcagg 20
<210> 14
   <211> 28
   <212> DNA
   <213> Artificial sequence
<220>
   <223> primer
<400> 14
   ggggggggat ccgacctttc tcttcttc 28
<210> 15
   <211> 26
   <212> DNA
   <213> Artificial sequence
<220>
   <223> primer
<400> 15
   ggggatccga gagagctgac gggcag 26
<210> 16
   <211> 26
   <212> DNA
   <213> Artificial sequence
<220>
   <223> primer
<400> 16
   ggtcatgaag tgctctgtga aattcg 26
<210> 17
   <211> 87
   <212> DNA
   <213> Artificial sequence
<220>
   <223> primer
<400> 17
   ggccatgggc ccccccgacc gatgtcagcc tgggggacgg ctgcacttag acggcgagga 60
   cgtggcgatg gcgcacgccg acgcgct 87
<210> 18
   <211> 26
   <212> DNA
   <213> Artificial sequence
<220>
   <223> primer
<400> 18
   ccgagctcct acccaccgta ctcgtc 26
<210> 19
   <211> 46
   <212> DNA
   <213> Artificial sequence
<220>
   <223> primer
<400> 19
   gatccatact gtatgagcat acagtatact gtatgagcat acagta 46
<210> 20
   <211> 46
   <212> DNA
   <213> Artificial sequence
<220>
   <223> primer
<400> 20
   gatctactgt atgctcatac agtatactgt atgctcatac agtatg 46
<210> 21
   <211> 29
   <212> DNA
   <213> Artificial sequence
<220>
   <223> primer
<400> 21
   ggagactcgc atgcgcaaga cccttcctc 29
<210> 22
   <211> 30
   <212> DNA
   <213> Artificial sequence
<220>
   <223> primer
<400> 22
   cccgggacta gttgtaattg taaatagtaa 30
<210> 23
   <211> 18
   <212> DNA
   <213> Artificial sequence
<220>
   <223> primer
<400> 23
   agcttggact agagcttg 18
<210> 24
   <211> 18
   <212> DNA
   <213> Artificial sequence
<220>
   <223> primer
<400> 24 18
   gatccaagct ctagtcca 18
<210> 25
   <211> 29
   <212> DNA
   <213> Artificial sequence
<220>
   <223> primer
<400> 25 29
   gaattcgagt ccaaaatgtt ggcatttag 29
<210> 26
   <211> 30
   <212> DNA
   <213> Artificial sequence
<220>
   <223> primer
<400> 26 30
   gaattcaagc ttttcgaaat caacgtttgt 30

## Description

### Field of the Invention:

The present invention relates to a method for detecting estrogen-like chemicals (including estrogen) using a transformed plant.

### Prior Art:

Many endocrine disrupting chemicals, commonly referred to as "environmental hormone", are not decomposed easily and are known to remain for long time in the environment. Since many environmental hormones affect intranuclear receptors and disrupt primary information transduction in living organism, they are suggested to detrimentally affect biological function, particularly to reproductive function and are feared for their effects on ecosystem.
It is known that steroid hormones transported by animal endocrine system generally bound to an intranuclear receptor in a target cell and control the expression of a target gene. Intranuclear receptors constitute a big gene family and contain conserved domain structure (A/B, C, D, and E). C domain relates to DNA binding and E domain relates to steroid hormone (ligand) binding. AF-2 (Activation Function 2) present in E domain is necessary to induce ligand-dependently transcriptional activation. Binding of an intranuclear receptor with a ligand enhances the interaction between AF-2 and transcriptional coactivator of p160 family and activates ligand-dependently the transcription of a target gene. An estrogen receptor belongs to the intranuclear receptor family, is activated by the binding with estrogen and activates the transcription of a target gene.

The development of biosensors for detecting estrogen-like chemicals has been universally conducted by the use of cells that express an intranuclear receptor and a reporter gene etc. (Reference 1 etc.). Although these biosensors are generally based on the enhancement of the expression of a reporter gene by binding of estrogen-like chemicals with an intranuclear receptor, the mechanism to express reporter gene after binding of estrogen-like chemicals with the intranuclear receptor is controversial.
For example, a method for detecting estrogen-like chemicals is reported by the use of a yeast two hybrid method, which is applied ligand-dependent interaction between ligand binding domain of estrogen receptor and a coactivator (Reference 2). However, the yeast two hybrid method needs sterilizing procedures and is feared for possible secondary pollution on the environment by organic solvent itself used for extraction.
Furthermore, a system without sterilizing procedures is designed, which transduces a plant (*Arabidopsis thaliana*) with a chimeraic gene comprising DNA binding domain, transcriptional activation domain and intranuclear receptor (Reference 3). Although this is preferable due to its simple design, the week point is its low sensitivity.
Moreover, a detection method by the use of a transformant transduced with ligand-dependent transcriptional factor, coactivator and reporter gene is designed (Reference 4).
Reference 1: Japanese patent publication2002-330759
Reference 2: Toxicology and Applied Pharmacology 154, 76-83 (1999)
Reference 3: The Plant Journal (2000) 24 (2), 265-273.
Reference 4: Japanese patent publication 2002-247986

### Problems to be solved by the Invention:

The purpose of the present invention is to provide an inexpensive, simple and easy method for detecting estrogen-like chemicals, which uses a plant, needs not use a complicated procedure such as sterilization and is highly sensitive as a sensor.

### Means to solve the Problems:

Enhanced transcriptional activation cannot be observed by the method that just express coactivator in a plant, such as the method in Reference 4. Moreover, the use of glucocorticoid receptor and estrogen receptor as it is, such as the method in Reference 3, results in rather insensitive sensor, since these are located usually outside the nucleus and translocated into nucleus after binding with a ligand.
In contrast, the method of the present invention involves transduction of effector 2 linked to a transcriptional activation domain with modified coactivator as well as effector 1 having an intranuclear receptor and DNA binding domain. Therefore, the method allows detecting a ligand by the use of ligand-dependent interaction with a coactivator, wherein the interaction arises under low concentration of ligand and is originated from interaction with a product of effector 1, allows improving the sensitivity of the sensor markedly.

Namely, the present invention is a transformed plant, which is transformed by transducing effector 1 sequence, effector 2 sequence, a target DNA and a reporter gene, wherein said effector 1 sequence has a promoter, a nuclear localization signal, and a chimeric gene comprising a polynucleotide domain coding a polypeptide binding to a target DNA and a polynucleotide domain coding a ligand binding polypeptide of intranuclear receptors for estrogen-like chemicals, said effector 2 sequence has a promoter, a nuclear localization signal, and a chimeric gene comprising a polynucleotide domain coding an interacting domain with intranuclear receptors in a transcriptional coactivator and a polynucleotide domain coding a transcriptional activation domain, and said reporter gene locates in the down stream of said target DNA.
Moreover, the present invention is a method for detecting an estrogen-like chemical, comprising the steps of contacting said transformed plant with an estrogen-like chemical and detecting the expression of said reporter gene in said plant.

### Brief Description of the Drawings:

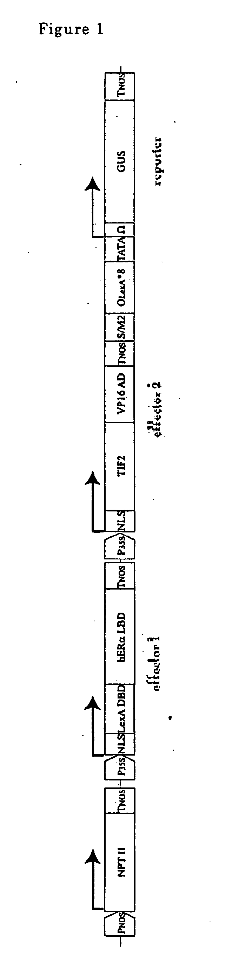

Figure 1 shows the structure of gene transduced to *Arabidopsis thaliana.* NPT II: neomycin phosphate-group transferase coding gene, NLS: nuclear localization signal of SV40 virus-derived T antigen: LexA DBD: DNA binding domain of *E. coli*-derived Lex A protein, hERα LBD: Ligand binding domain of human-derived estrogen receptor, TIF2: human-derived transcriptional coactivator TIF2 coding gene, VP16 AD: transcriptional activation domain of VP16 protein of herpes simplex virus, GUS: *E. coli*-derived β-gulcuronidase gene coding domain, Pnos: a promoter of soil bacterium-derived nopaline synthetase, P35S: 35S RNA promoter of cauliflower mosaic virus, Tnos: soil bacterium-derived transcriptional termination sequence, O_{LexA}*8: Eight consecutive DNA target sequence of LexA protein, S/M2: tobacco-derived nuclear matrix interaction DNA domain II, Ω: translational activation DNA sequence.

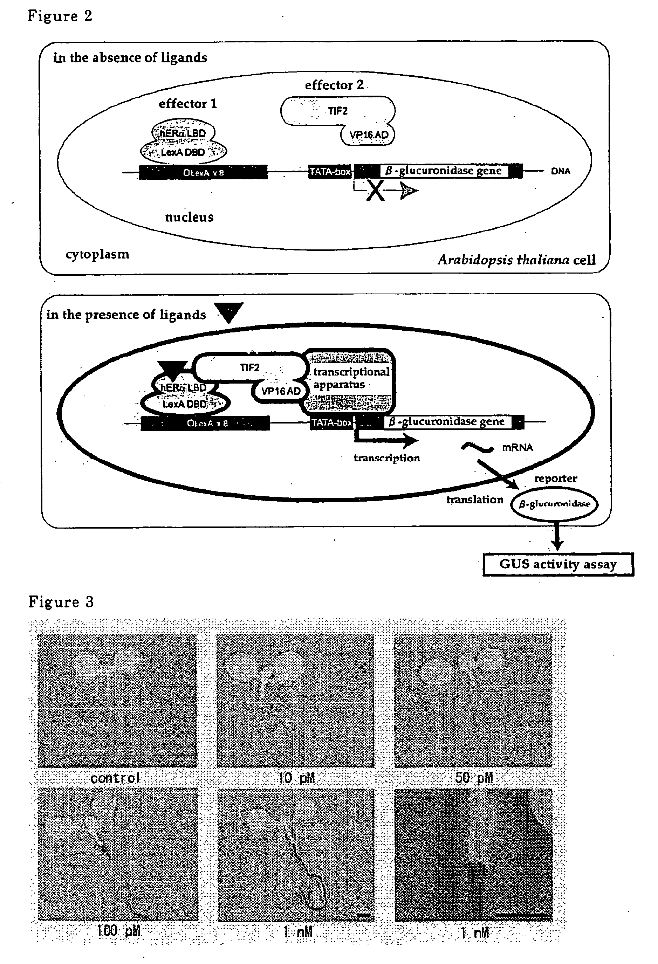

Figure 2 shows molecular mechanism of estrogen detection by the plant two effector system. A chimeric estrogen receptor comprising hERα LBD and LexA DBD, expressed steadily in nucleus, bounds to OLexA located in upstream of GUS gene, a reporter gene. Binding of estrogen to hERαLBD induces ligand-dependent interaction with chimeric transcriptional coactivator including TIF2 and VP16AD. The interaction allows to transmit transcriptional activation signal of VP16AD to plant transcriptional apparatus, which induces transcription and translation of GUS gene. Estrogen detection is possible by measurement of enzyme activity of GUS protein.
Figure 3 shows the change of 17β-estradiol dose-dependent reporter gene expression. The bar in the figure exhibits 1 mm.
Figure 4 shows the change of 17B-estradiol dose-dependent reporter gene expression. The ordinate shows GUS specific activity (pmols 4-MU h⁻¹ mg protein ⁻¹). The error was calculated based on three independent experiments.
Figure 5 shows the variation of 17β-estradiol exposed day-dependent reporter gene expression. The ordinate shows GUS specific activity (pmols 4-MU h⁻¹ mg protein⁻¹). The error was calculated based on three independent experiments.
Figure 6 shows response of reporter gene against various estrogen-like chemicals. (A) p-n-nonylphenol (NP), (B) diethylstilbestrol (DES), (C) bisphenol A (BPA), (D) genistein are used as estrogen-like chemicals. The ordinate shows GUS specific activity (pmols 4-MU h⁻¹ mg protein ⁻¹). The error was calculated based on three independent experiments.

### Detailed Description of the Invention:

Figure 2 shows the contour of detecting chemicals with estrogen activity by the use of the transformed plant of the present invention. The gene transduced in the transformed plant is constructed by two kinds of effector genes comprising chimeric estrogen receptor and chimeric transcriptional coactivator expressed steadily in plant cells and a reporter gene (e.g. β-glucuronidase) transcriptionally activated by ligand-dependent interaction between effector gene products. Highly sensitive detection of chemicals with estrogen activity in test samples is accomplished by measuring the activity of a reporter gene product produced in a transformed plant by estrogen-like chemicals (ligands).

"Effector 1 sequence" has a promoter, a nuclear localization signal, and a chimeric gene comprising a domain coding a polypeptide binding to a target DNA and a domain coding a ligand binding polypeptide of intranuclear receptors for estrogen-like chemicals. Effector 1 sequence may appropriately contain a domain coding all or a part of other intranuclear receptors derived from eukaryotes.
"Promoter" includes plant virus-derived 35S promoter, plant related bacterium-derived nopalin synthase promoter and other natural or synthetic promoters functional in other plant cells.
"Nuclear localization signal" includes polypeptide sequence (e.g. nuclear transport signal of T antigen of SV40 virus) recognized by proteins directly related to transportation to nucleus and polypeptide sequence necessary for transportation indirectly via protein with nuclear transport signal.

"The domain coding a polypeptide binding to a target DNA" is a polypeptide domain binding to a target DNA, and "target DNA" involves various straight polynucleotide sequence more than 4 bases. The binding domain to the target DNA is polypeptide domain recognizing and specifically binding to the target DNA sequence.

"Estrogen-like chemicals" in the present invention involve both estrogen and estrogen-like chemicals and such chemicals involve chemicals described in Table I (to the end of the description, Nishihara et al., J. Health Science. 46(4): 282-298, 2000).
"The domain coding a ligand binding polypeptide of intranuclear receptors for estrogen-like chemicals" is a domain containing polypeptide sequence accompanied with binding activity to eukaryote-derived estrogen.
"Chimeric gene comprising a nucleotide domain coding a polypeptide binding to a target DNA and a nucleotide domain coding a ligand binding polypeptide of intranuclear receptors for estrogen-like chemicals" is composed by the linkage of polynucleotides coding the polypeptide containing these.

"Effector 2 sequence" has a promoter, a nuclear localization signal, and a chimeric gene comprising a domain coding an interacting domain with intranuclear receptors in a transcriptional coactivator and a domain coding a transcriptional activation domain. Effector 2 sequence may appropriately contain polynucleotide domain coding a transcriptional inactivation domain as well as said chimeric genes.
"Promoter" and "nuclear localization signal" may be selected from or may be the same as the promoters and polypeptides defined for said effector 1 sequence.

"A transcriptional coactivator" involves polypeptides etc., which functionally increase transcriptional activity by interacting with eukaryote-derived intranuclear receptors including CBP/p300 and p160 family proteins (TIF2, SRC1, ACTR, TRAP220, TIP60) etc.
"The interacting domain with intranuclear receptors in a transcriptional coactivator " involves polypeptide domain referred to as NID and accompanied with LXXLL (L=leusine) motif.
"A transcriptional activation domain" is a polypeptide domain, which activates transcription by interacting with fundamental transcriptional factors and transcriptional apparatus such as polypeptides containing a lot of basic amino acids in a chain structure and polypeptide domain containing a lot of glutamic acid in a chain structure.
"Chimeric gene comprising a domain coding an interacting domain with intranuclear receptors in a transcriptional coactivator and a domain coding a transcriptional activation domain " is composed by the linkage of polynucleotides coding the polypeptide containing these.

"Target DNA" is the polynucleotide recognized and bound specifically by a DNA binding protein and could be the polynucleotides linked more than two units together. The sensitivity of detecting estrogen is increased by tandem connection of said target DNA rather than by the use of isolated target DNA, since the frequency of binding of chimeric estrogen receptor with the DNA sequence is increased.
"Reporter gene" involves fluorescent proteins, enzyme with measurable activity and polynucleotide domain coding a protein except enzymes with measurable activity. The reporter gene locates at the down stream of the target DNA.

The reporter gene expression domain could be linked with S/MII
sequence (polynucleotide sequence with affinity to nuclear scaffold) to stabilize gene expression in a plant, Omega sequence (polynucleotide sequence coding a sequence on mRNA augmenting translation efficiency) to increase protein synthesis in a plant, TATA BOX etc. as well as the above target DNA and a reporter gene. In addition to them, a polynucleotide sequence affecting the efficiency of transcription and translation etc. could be suitably linked if necessary.

The transformable plants used for the present invention are *Arabidopsis thaliana,* tobacco, tomato, *Antirrhinum majus,* potato, dicotyledon such as legume etc. and monocotyledon such as *Zea mays,* poaceous plant and wheat etc.
Use of plants as detection method like present invention has a lot of advantage. Since plant absorbs environmental chemicals through a root, there is no need to isolate and purify chemicals from samples and to use sterilizing procedures. Furthermore, seeds could be stably stored for a long time, do not require a special apparatus to store and hence are less costly on storing the sensor than other bioassay method. Moreover, an *Arabidopsis thaliana* plant, for example, generates about 2000 seeds and hence mass production allows the measurement to be less costly.

Said plants are transduced with said effector 1 sequence, effector 2 sequence, target DNA and reporter genes. The transduction procedure involves flower dipping method via soil bacterium (e.g. Agrobacterium), electroporation method, microprojectile bombardment method and microinjection method etc.

A method for contacting transformed plant with estrogen-like chemicals involves contact following seeding on a water/soil/agar medium containing estrogen. Or an individual plant, which was seeded and grown on a water/ soil/agar medium without estrogen, may be transplanted to a water/soil/agar medium containing estrogen and contacted with estrogen.

Methods for detecting reporter gene may be different depending on the reporter gene. In case that fluorescent protein is used as a reporter gene, for example, fluorescent microscope and fluorescent colorimeter could be used to detect. In case that enzyme with a measurable enzyme activity is used as a reporter gene, a method depending on enzyme activity measurement may be used. Also, in case that protein without a measurable enzyme activity is used as a reporter gene, observation of the change of physiological function and morphology of plant due to the expression of the activity could be used.
The following Examples further illustrate the present invention, but it is not intended to limit the scope of the invention.

### Example 1

In this Example, a transformed plant was prepared by transduction of a gene shown in Fig. 1.
Chimeric estrogen receptor containing hERαLBD and LexA DBD, and chimeric transcriptional coactivator including TIF2 NID and VP16 AD are constantly excessively expressed in a transformed *Arabidopsis thaliana* plant owing to cauliflower mosaic virus (CaMV) 35S promoter controlling strongly the transcription of the genes located at the down-stream of the promoter in the plant. Said two kinds of chimeric proteins contain nuclear localization signal of SV40 virus-derived T-antigen and constantly localized in a nucleus. Chimeric estrogen receptor binds to a target sequence (OLexA) of LexA protein located at the up-stream of GUS gene, i.e. a reporter gene, in nucleus. Binding of estrogen with hERαLBD in chimeric extrogen receptor induces change of protein structure of hERαLBD and allows to interact with TIF2 NID portion of chimeric transcriptional coactivator. The interaction transfers the transcriptional activation signal of VP16AD derived from herpes simplex virus fused to TIF2ND to transcriptional apparatus of the plant and induces transcription and translation of GUS gene estrogen-dependently. It is possible to detect estrogen by measuring enzyme activity of GUS protein in the transformed plant.

Firstly, a gene was constructed for transduction into *Arabidopsis thaliana.* Effector 1 gene coding chimeric estrogen receptor was prepared by the integration of the linkage of a gene coding the polypeptide connecting nuclear localization signal (NLS) of T-antigen of SV40 virus, LexA DNA binding domain (LexA DBD) and ligand binding domain (hERαLBD) of human estrogen receptor α, and a terminator (TNOS) of nopalin synthase gene of agrobacteria into the down-stream of cauliflower mosaic virus 35S promoter (P35S). Effector 1 gene construction was prepared by PCR amplification of a fragment (P35S-NLS) coding from P35S to NLS, a fragment coding LexA DBD, a fragment coding hERαLBD and a fragment of TNOS, separately.
P35S-NLA fragment was prepared by the use of 35S-NLS-GFP (Gifted from Dr. Niwa Yasuo, University of Shizuoka, Department of Food and Nutritional Science, Yada 52-1, Shizuoka-city, Curr Biol, 6:325·30 (1996)) as a template, and 5'-GGAAGCTTGCATGCTGCAGG-3' (SEQ ID NO: 1) and 5'-GGGCTAGCGACCTTTCTCTTCTTCTT-3' (SEQ ID NO: 2) as primers by PCR reaction in a way that a HindIII site and a Nhel site are present at the 5' side and the 3' side, respectively.

LexA DBD fragment was prepared by the use of *E. coli* genome as a template, and 5'-GGGCTAGCATGAAAGCGTTAACGCCC-3' (SEQ ID NO: 3) and 5'-GGGCCGGCCTGGTTCACCGGCAGCCAC-3' (SEQ ID NO: 4) as primers by PCR reaction in a way that a NheI site and a Fsel site are present at the 5' side and the 3' side, respectively, of the domain coding 87 amino acids of amino acids 1-87 of Lex A (SEQ ID NOs: 5 and 6), i.e. a repressor of *E. coli.*
hERαLBD fragment was amplified after separated to N terminal fragment coding 43 amino acids of amino acids 281-323 of hER (Gen Bank: NM_0000125) and C terminal fragment coding 272 amino acids of amino acids 324-595 of hER and a termination codon.
Both fragments were prepared by PCR reaction by the used of human ovary cDNA library as a template and N terminal fragment was prepared by PCR reaction by the use of
GGGGCCGGCCGTCTGCTGGAGACATGAGA-3' (SEQ ID NO:7) and
GGGCTCAGCATCCAACAAGGCACTGAC-3' (SEQ ID NO:8) as primers in a way that a FseI site and a Bpu11021site are present at the 5' side and the 3' side, respectively.
C terminal fragment was prepared by PCR reaction by the use of
5'-GGGCTGAGCCCCCCATACTCTATTCCGAGTATGATCCTACCAGACCCTTCA GTGAGGCTT-3' (SEQ ID NO:9) and
5'-GGGCGGCCGCTCAGACTGTGGCAGGGAA-3' (SEQ ID NO:10) as primers in a way that a Ppu11021 site and a NotI site are present at the 5' side and the 3' side, respectively, after disruption of an internal Hind III site by a base substitution.

TNOS fragments were prepared by the use of pBI221 (CLONTECH, Palo Alto, CA, USA) as a template, and 5'-GGGCGCGCCGCGAATTTCCCCGATCGTTC-3' (SEQ ID NO: 11) and 5'-GGGAATTCACTAGTCCGATCTAGTAACATAGA-3' (SEQ IDNO: 12) as primers by PCR reaction in a way that a NotI site and a EcoRI site are present at the 5' side and the 3' side, respectively.
Effector 1 plasmid was prepared by the insertion of the above 4 fragments into HindIII/EcoRI site of pBI221 (BD Biosciences Clontech, Palo Alto, CA, USA).

Effector 2 gene coding chimeric coactivater was prepared by the integration of the linkage of a gene coding a polypeptide connecting nuclear localization signal (NLS) of T-antigen, a part of human transcriptional coactivater (hTIF2 NID) (human transcriptional intermediately factor 2 nuclear receptor interaction domain) and transcriptional activation domain (VP16 AD) of herpes simplex virus VP16, and TNOS into the down stream of P35S.
Effector 2 gene construction was prepared by PCR amplification of a fragment coding from P35S to NLS, a fragment coding hTIF2 NID and a fragment coding VP16 AD, separately.

P35S-NLS fragment was prepared by the use of 35S-NLS-GFP as a template and 5'-GGAAGCTTGCATGCTGCAGG-3' (SEQ ID NO: 13) and 5'-GGGGGGGGATCCGACCTTTCTCTTCTTC-3' (SEQ ID NO:14) as primers by PCR amplification in a way that a HindIII site and a BamHI site are present at the 5' side and the 3' side, respectively.
hTIF2 NID fragment was prepared by the use of human liver cDNA library as a template and 5'-GGGGATCCGAGAGAGCTGACGGGCAG-3' (SEQ ID NO: 15) and 5'-GGTCATGAAGTGCTCTGTGAAATTCG-3' (SEQ ID NO: 16) as primers by PCR reaction in a way that a BamHI site and a BspHI site are present at the 5' side and the 3' side, respectively, of the domain coding 246 amino acids of amino acids 624-869 of hTIF2.

VP16 AD fragment was prepared by the use of herpes simplex virus transcriptional activator VP16 gene (GenBank M15621, Clontech, Palo Alto, CA, USA) as a template and 5'-GGCCATGGGCCCCCCCGACCGATGTCAGCCTGGGGGACGGCTGCACTTAGACG GCGAGGACGTGGCGATGGCGCACGCCGACGCGCT-3' (SEQ ID NO: 17) and 5'-CCGAGCTCCTACCCACCGTACTCGTC-3' (SEQ ID NO: 18) as primers by PCR reaction in a way that a NcoI and a SacI site are present at the 5' side and the 3' side, respectively, of the domain coding 78 amino acids of amino acids 413-490 of VP16 and a termination codon. During the preparation, an internal SalI site was disrupted by a base substitution.
Effector2 plasmid was prepared by the consecutive insertion of the above three fragments into HindIII-SacI site of pBI221

The reporter gene coding glucuronidase is prepared by the connection of translation amplification sequence (Ω sequence), a full length of gene coding E. coli glucuronidase and TNOS at the down-stream of the chimeric promoter combined a target DNA sequence of LexA protein (eight times repeated sequence) with TATA sequence of P35S. Reporter gene was constructed followed by the preparation of octamer fragment of LexA target sequence, TATA-Ω fragment from TATA box of P35S to Ω sequence and HindIII-BamHI linker.
As for the LexA target sequence, a dimer fragment of LexA target sequence located between BamHI and BglII restriction enzyme sites was prepared by annealing of 5'-GATCCATACTGTATGAGCATACAGTATACTGTATGAGCATACAGTA-3' 'SEQ ID NO: 19) and 5'-GATCTACTGTATGCTCATACAGTATACTGTATGCTCATACAGTATG-3' (SEQ ID NO:20).
Four copies of said sequence were connected in tandem in a way that a BamHI site and a BglII site were present at the 5' side and the 3' side, respectively, of the octamer fragment of LexA target sequence.

TATA-Ω fragment was prepared by the use of 35S-NSL-GFP as a template and 5'-GGAGACTTCGCATGCGCAAGACCCTTCCTC-3' (SEQ ID NO: 21) and 5'-CCCGGGACTAGTTGTAATTGTAAATAGTAA-3' (SEQ ID NO: 22) as primers by PCR reaction in a way that a BglII site and a SmaI site are present at the 5' side and the 3' side, respectively.
HindIII-BamHI linker was prepared by annealing single-stranded oligo DNA 5'-AGCTTGGACTAGAGCTTG-3' (SEQ ID NO: 23) with 5'-GATCCAAGCTCTAGTCCA-3' (SEQ ID NO: 24) to double-strand. The reporter plasmid was prepared by consecutive insertion of said three fragments into HindIII-SmaI site of pBI221 (BD Biosciences Clontech, Palo Alto, CA, USA).

For the stabilization of the expression of the reporter gene in a transformed plant, Scaffold/Matrix attatchment regions II (S/M II), ie. a Matrix attatchemnt region of tobacco (Nicotiana tabacum), was inserted to 5' side of octamer fragment of LexA target sequence. For the above purpose, a DNA fragment was prepared by the use of tobacco genome DNA as a template and 5'-GAATTCGAGTCCAAAATGTTGGCATTTAG-3' (SEQ ID NO: 25) and 5'-GAATTCAAGCTTTTCGAAATCAACGTTTGT-3' (SEQ ID NO: 26) as primers by PCR reaction in a way that an EcoRI site is present at the 5' side and a HindIII and an EcoRI sites were present the 3' side of S/MII.
S/M II plasmid was prepared by the insertion of the fragment into pGEM-T vector (Promega Corp., Madison, WI, USA)

A plasmid (hereinafter referred to as two effector plasmid) containing two effector genes and a reporter gene was prepared by the insertion of effector 1 fragment (HindIII-EcoRI fragment), effector 2 fragment (HindIII-EcoRI fragment), S/MII fragment (EcoRI-HindIII fragment), obtained by the treatment of each prepared plasmids with restriction enzymes, into HindIII site of reporter plasmid.

Then, the above prepared two effector plasmid was transduced into *Arabidopsis thaliana.* Columbia strain (wild strain) of *Arabidopsis thaliana* was used for the preparation of transfromed plant. After agrobacteria C58 strain was transduced with two effector plasmid by electroporation, the agrobacteria was infected to *Arabidopsis thaliana* to transform the plant. The seed (T1 seed) obtained from the transformed *Arabidopsis thaliana* was seeded into MS agar plate containing 1% sucrose, 0.8% plant agar, 50 µg/ml Kanamycin, 100 µg/ml Claforan and was selected for the transformants containing transduced genes. T2 seed was obtained from Kanamycin resistant T1 plant. T3 seed was obtained from T2 plant.

### Example 2

In this Example, estrogen dose-dependent expression of GUS gene in transformed *Arabidopsis thaliana* obtained in Example 1 was examined.
Seeds of T3 were sown on a MS agar medium containing 17β-estradiol (WAKO Pure Chemical Inds.). The expression of GUS gene was examined by GUS activity staining method and GUS activity assay method by the use of a transformed plant grown for a week.

The induction of reporter gene expression by estrogen in transformed *Arabidopsis thaliana* was performed by the following procedures. Seeds of transformed *Arabidopsis thaliana* were sown on a MS agar medium containing estrogen (17β-estradiol) (1/10000 volume of estrogen dissolved in DMSO (WAKO Pure Chemical Inds.) was added) and left in a dark place at 4°C for 3 days. After that the transformed *Arabidopsis thaliana* was sprouted and grown in a bright place at 22°C for specified days for the continuous exposure to estrogen.

GUS activity staining method was performed in the following way. The transformed *Arabidopsis thaliana* exposed to estrogen was treated with GUS staining. The plant was immersed in a staining buffer (2 mM 5-bromocloroindolyl-β-D-gluclonide (X-Glue), 50 mM Sodium phosphate buffer (pH 7.0), 10 mM EDTA, 0.1% TritonX-100, 0.5 mM ferricyanide; 0.5 mM ferrocyanide) and left under reduced pressure for 15 min in a desiccator. Then, the plant was allowed to stand for 3 hr at 37°C, was removed chlorophyll in 70% ethanol and was observed. The result is shown in Fig.3.
Gus staining was performed for the whole plant and development of indigoblue color, which is obtained by decomposition of X-Gluc (i.e. a substrate) by GUS protein, was observed at the root part of the transformed plant in the presence of more than 0.05 nM estrogen. The part, where GUS color was observed, is the part, where the plant was directly contacted with an agar medium, and it is interpreted that estrogen is infiltrated into the plant by diffusion from the medium.

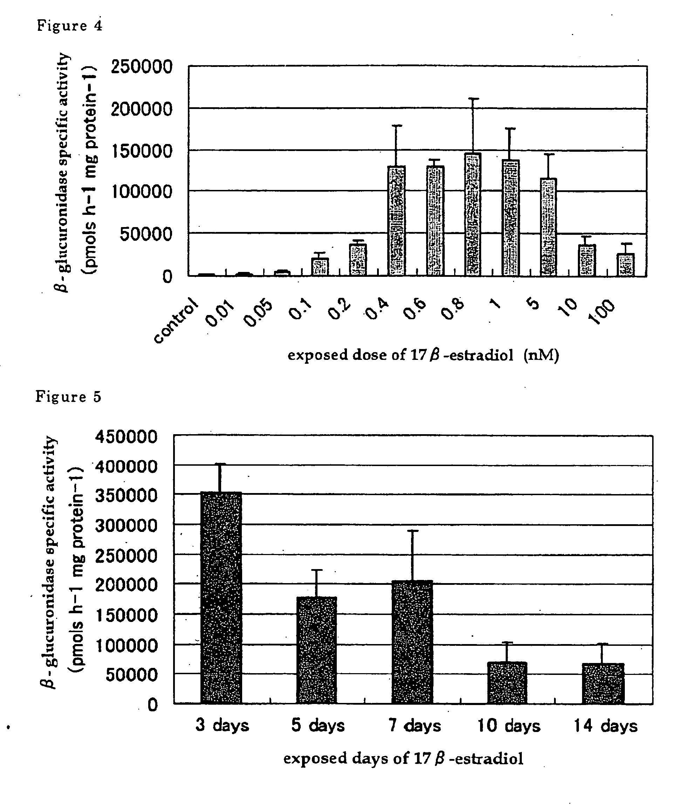

The Gus activity assay method was performed in the following way. Gus activity in the protein extracted form the transformed *Arabidopsis thaliana* exposed to estrogen was measured. Twenty plants treated with estrogen were frozen in liquid nitrogen and were crushed into powder by a homogenizer. Then, the extraction buffer (50 mM NaH₂PO₄, 10 mM EDTA, 0.1% Triton-X-100, 0.1% N-sodium lauroyl sarcosinate, 10 mM 6-mercaptoethanol) was added and soluble protein was extracted. 4-methylumbelliferyl-β-D-glucronide (4-MUG) was used for a substrate of GUS activity assay. The extracted protein solution was added with extraction buffer containing final 1 mM 4-MUG and was reacted at 37°C for 1 hr. The enzymatic reaction was stopped by the addition of 0.2 M Na₂CO₃ after 1 hr. The concentration of 4-MU released from 4-MUG by the catalytic action of GUS was measured by a fluorescent spectroscopy (Hitachi F-4500, 455 nm fluorescence excited by 365 nm light was measured.). Also, protein concentration in the solution was measured and measured values obtained by fluorescent spectroscopy were standardized by the correction depending on the total protein in the protein solution. The results are shown in Fig. 4.

The expression of GUS gene in the transformed plant increased in the 17β-estradiol concentration range from 0.05 nM to 0.8 nM and decreased in the concentration beyond 5 nM.

### Example 3

In this Example, the change of the activity of reporter gene depending on the exposed days to the transformed *Arabidopsis thaliana* was examined. The induction of reporter gene expression in the transformed *Arabidopsis thaliana* by estrogen was performed as in Example 2.
T3 seed were sown on a MS agar medium containing 17β-estradiol and grown for 3-14 days. After harvest of the grown transformants, the expression of GUS gene was examined by the GUS activity assay method. The results are shown in Fig. 5.
The activity of GUS gene is shown to decrease as exposed days increased.

### Example 4

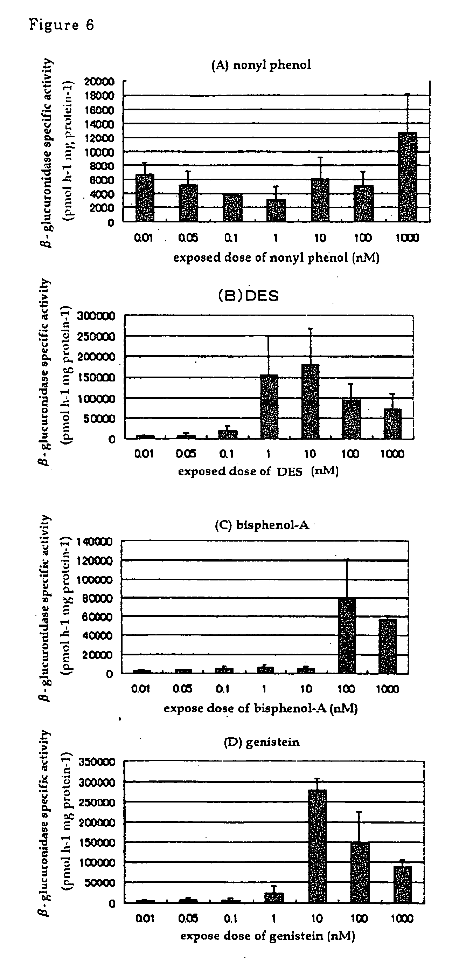

In this Example, the response of the transformed *Arabidopsis thaliana* was examined for chemicals with estrogen agonist activity. The induction of reporter gene expression in the transformed *Arabidopsis thaliana* by estrogen was performed as in Example 2.
T2 seeds, i. e. the second generation of the transformants, was sown on a MS agar medium containing various concentration of estrogen-like chemicals such as diethylstilsbestrol (DES, ICN Biomedicals Inc. CT, USA), p-n-nonylphenol (NP, WAKO Pure Chemical Inds.,), bisphenol A (BPA, WAKO Pure Chemical Inds.,) and genistein (EXTRASYNTHESE S. A. Genay, France) and grown for a week. The expression of GUS gene in the transformed plant was examined by the GUS activity assay method. The results are shown in Fig 6 (A) to (D).

DES shows an expression profile responding from 0.1 nM. Also, NP, BPA and genistein show increased expression of GUS gene at more than 1000 nM, 100 nM and 1 nM, respectively. The optimal concentration of DES, BPA and genistein were 10 nM, 100 nM and 10 nM, respectively for the expression of GUS gene. NP shows increase in the expression of GUS gene from 1000 nM.

### Industrial Field of the Invention

The seeds of the transformed plant of the present invention, which were suspended in water containing estrogen-like chemicals and were budded and grown under light irradiation, show the same sensitivity to estrogen as they were grown in an agar medium containing estrogen. Therefore, estrogen concentration could be measured by a very simple procedure as immersion of seeds into the sample solution, if a test sample is aqueous solution. Furthermore, change of receptor used allows developing the assay system of other many environmental hormones, since coactivator could interact with many intranuclear receptors. Since the concentrations of environmental hormones in environment are extremely low and the sensitivity of the assay method of the present invention is high, the construction of the reporter assay system was accomplished.

**Table 1**

| |
|---|
| 1,1,1,2-Tetrachloroethane, 1,2,3-Trichlorobenzene, 1,2,3-Trichloroethane, 1,2,3-Trimethylbenzene, 1,2,4,5-Tetramethylbenzene, 1,2,4-Trichlorobenzene, 1,2,4-Trimethylbenzene, 1,2-Dibromoethane, 1,2-Dichlorobenzene, 1,2-Diethylbenzene, 1,2-Dimethylnaphthalene, 1,2-Dinitrobenzene, 1,2-Epoxyethlbenzene, 1,3,5-Triethyltoluene, 1,3,5-Trimethylbenzene, 1,3,5-Triphenylcyclohexane, 1,3-Dichloro-2-propanol, 1,3-Dichloropropene,mixture, 1,3-Diphenylpropane, 1,4-Dichlorobenzene, 1,4-Diethylbenzene, 1,4-Dioxane, 1,6-Dinitropyrene, 1,8-Dimethylnaphthalene, 1,8-Dinitropyrene, 1.2-Dibromo-3-chloropropane, 10-Hydroxy benzo[a]pyrene, 11-Hydroxy benzo[a]pyrene, 12-Hydroxy benzo[a]pyrene, 17a-Estradiol, 17a-Ethynylestradiol, 1a-Phenyl-4a-(1'phenylethyl)tetralin, 1a-Phenyl-4e-(1'phenylethyl)tetralin, 1-Butanol, 1-Chloro-2,4-dinitrobenzene, 1-Chloro-2-nitrobenzene, 1e-Phenyl-4a-(1'phenylethyl)tetralin, 1e-Phenyl-4e-(1'phenylethyl)tetralin, 1e-Phenyl-4e-(1'-phenylethyl)tetralin, 1-Hydroxy pyrene, 1-Methylnaphthalene, 1-Nitropyrene, 1-Nonanol, 1-Tridecanol, 2,2',2''-Nitrilotriethanol, 2,2-Bis(3,5-dibromo-4-hydroxyphenyl) propane, 2,2'-Dihydroxybiphenyl, 2,4,5-Trichlorophenol, 2,4,5-Trichlorophenoxyacetic acid, 2,4,6-Triphenyl-10hexene, 2,4-Diaminotoluene, 2,4-Diaminotoluene, 2,4-Dichloroaniline, 2,4-Dichlorophenoxyacetic acid, 2,4-Dinitroaniline, 2,4-Diphenyl-butane, 2,5-Dichloroaniline, 2,6-Dimethylnaphtalene, 2^Aminoethanol, 2-Aminoanthracene, 2-Aminoanthraquinone, 2-Aminotoluene, 2-Butoxyethyl phthalate, 2-Chloro-1,1,2-trifluoroethyl ethyl ether, 2-Ethoxyethanol, 2-Ethyltoluene, 2-Hydroxy enzo[a]pyrene, 2-Hydroxy fluorine, 2-Hydroxydibenzofuran, 2-Hydroxyethyl methacrylate 2-Mercaptobenzothiazole, 2-Mercaptoimidazoline, 2-Methyl-1-propanol, 2-Methylphenol, 2-Methylpyridine, 2-Nitrofluorene, 2-Nitrophenel, 2-Phenylene diamine, 2-sec-Butylphenol, 2-tert-Butylphenol, 3,3',4,4',5-Pentachlorobiphenyl(PCB 126), 3,4-Dichlorophenol, 3,8-Dihydroxy-2,8-dichlorodibenzofuran, 3-Aminophenol, 3-Ethyltoluene, 3-Methylcholanthrene(MC), 3-Nitrofluoranthene, 3-Nitrophenol, 3-tert-Butylphenol, 4-(branched)-Nonylphenol, 4,4'-Dihydroxybenzophenone, 4,4'-dihydroxybiphenyl, |
| 4,4'-Thiobiphenyl, 4-Acryloyloxyethyl trimeritic acid, 4-Acryloyloxyethyl trimeritic acid , nhydrate, 4-Amino butylbenzoate, 4-Aminobenzoic acid, 4-Aminobenzoic acid diglucoside, 4-Bromophenol, 4-Chloro-3,5-xylenol, 4-Chloro-3-methylphenol, 4-Chloroaniline, 4-Chlorophenol, 4-Choronitrobenzene, 4-Chorortoluene, 4-Ethylphenol, 4-Hydroxy benzo[a]pyrene, 4-Hydroxy-2',3,5,5'-tetrachlorobiphenyl, 4-Hydroxy-2',4',6'-trichlorobiphenyl, 4-Hydroxy-4'-monochlorobiphenyl, 4-Hydroxyacetophenone, 4-Hydroxybenzaldehyde, 4-Hydroxybenzoic acidm, 4-Hydroxybiphenyl, 4-Hydroxy-tamoxifen,4-iso-Propyl-3-methylphenol, 4-Methacryloyloxyethl trimeritic acid, 4-Methacryloyloxyethl trimeritic acid anhydrate, 4-Methylphenol, 4-n-Butylphenol, 4-n-Heptylphenol, 4-n-Hexylphenol, 4-Nitroquinoline-N-oxide, 4-Nitrotoluene, 4-n-Nonylphenol, 4-n-Octylphenol, 4-Nonylphenol polyethyoxylate(10), 4-Nonylphenol polyethyoxylate(15), 4-Nonylphenol polyethyoxylate(2), 4-Nonylphenol, olyethyoxylate(23), 4-Nonylphenol polyethyoxylate(5), 4-n-Pentylphenol, 4-n-Propylphenol, 4-sec-Butylphenol, 4-tert-Butylbenzoic acid, 4-tert-Butylphenol, 4-tert-Octylphenol, 4-tert-Octylphenol, 4-tert-Octylphenol polyethoxylate(10), 4-tert-Octylphenol, polyethoxylate(15), 4-tert-Octylphenol polyethoxylate(2), 4-tert-Octylphenol, polyethoxylate(23), 4-tert-Octylphenol polyethoxylate(5), 4-tert-Pentylphenol, 4-Toluenesulfonamide, 5-Hydroxy benso[a]pyrene, 6-Hydroxy benso[a]pyrene, 6-Hydroxy-3,4-dichlorodibenzofuran, 7-Hydroxy benzo[a]pyrene, 7-Hydroxy-1,2,3,6,8-pentachlorodibenzofuran, 7-Hydroxy-3,4-dichlorodibenzofuran, 8-Hydroxy enzo[a]pyrene, 8-Hydroxy-2,3,4-trichlorodibenzofuran, 8-Hydroxy-2-menochlorodibenzofuran, 8-Hydroxy-3,4,6-trichlorodibenzofuran, 8-Hydroxy-3-monochlorodibenzofuran, 8-Hydrozy-3,4-dichlorodibensofuran, 9-Hydroxy benso[a]pyrene, 9-Hydroxy fluorine, 9-Hydroxy-2,6-dichlorodibenzofuran, 9-Hydroxy-3,4-dichlorodibenzofuran, Acephate, Acetaldehyde, Acetamide, Acetyleugenol, Acylamide, Adipic acid, Aflatoxin B1, a-Hexachlorocyclohexane, Alachlor, Aldicarb, Aldrin, a-Methylstyrene, Amitrole, Aniline, Anthracene, Antimony(III) chloride, Apigenin, Aplysiaterpenoid A, Atrazine, Benz[a]antharacene, Benzaldehyde, Benzalkonium chloride, Benzo[a]pyrene, Benzo[b]fluoranthene, Benzo[e]pyrene, Benzo[g,h,i]perylen, Benzo[k]fluoranthene, Benzoepinesulfate, Benzoic acid, Benzophenone, Benzylalcohol, Benzylbutyl phthalate, |
| b-Estracliol-17-acetate, b-Hexachlorocyclohexane, Bifenox, Biochanin A, Biphenyl, Bis(2-chloroethyl)ether, Bis(4-hydroxypheny)methane, Bis(4-hydroxyphenyl)sulfone, Bisphenol A, Bisphenol-Abischloroformate, Bisphenol-A-diglycidyl, ether, Bisphenol-A-dimethacrylate, Bisphenol-A-ehoxylate, Bisphenol-A-ehoxylate diacrylate, Bisphenol-A-proxylate, Bisphenolo-A-bischloroformate, Bisphenolo-A-diglycidyl ether, Bisphenolo-A-dimethacrylate, Bisphenolo-A-ethoxylate, Bisphenolo-A-ethoxylate diacrylate, Bisphenolo-A-proxylate, Boric acid, BPMC, Bromobutide, Bromodichloromethane, Bromoform, b-Sitosterol, Butylated hydroxyanisole, Butylated hydroxytoluene, Cadmium chloride, Campharquinone, Captans, Caravacrol, Carbaryl, Carbendazim, Carbofuran, Catechol, Chinomethionat, Chlorhexidine gluconate, Chlornitrofen, Chlorobenside, Chlorobenze, Chlorobenzilate, Chlorodibromomethane, Chloropropham, Chlorothalonil, Chlorpyrifos-methyl, cis-1,2-Diphenylcyclobutane, cis-Stilbene, Clomiphene, compound, Copper(II) sulfate, Coumaric, acid, Coumestrin, Coumestrol, Cucumechinoside D, Cumene, Cyanazin, Cyclohexanol; Cyclohexanone, Cyclohexylamine, Cyclosporin A, Cyfluthrin, Cyhalothrin, Cypermetrin, Daidzein, Daidzin, Dethyl adipate, Dexamethasone, Di-2-ethylhexyl adipate, Di-2-ethylhexyl phathalate, Diazinon, Dibenz[a,h]anthracene, Dibenzyl ether, Dibutyl adipate, Dibutyl phthalate, Dichlorvos, Dicloran, dicyclohexyl phthalate, Dicyclohexylamine, Dicyclopentadiene, Didecyldimethylammmonium , hloride, Dieldrin, Diethoxyleneglycol dimethacrylate, Diethyl phthalate, Diethyl sulfate, Diethylbenzene, mixture, Diethylene glycol, Diethylstilbesterol, Diflubenzuron, Diheptyl, phthalate, Dihidroglycitein, Dihydrogenistein, Dihydrotestosterone, Di-iso-butyl adipate, Di-iso-butylphathalate, Di-iso-decyl phathalate, Di-iso-nonyl phthalate, Di-iso-octyl, phthalate, Di-iso-propyl adipate, Di-iso-prppyl phthalate, Dimepiperate, Dimethoate, Dimethyl adipate, Dimethyl phthalate, Di-n-butyl phthalate, Di-n-hexyl phthalate, Di-n-pentyl phthalate, Di-n-propyl phthalate, Diphenyl carbonate, Diphenylamine, Diphenylmethane, Dipropyl phthalate, Dodecyl polyethoxylate(15), Dodecyl polyethoxylate(3), Dodecyl polyethoxylate(5), Dodecyl polyethoxylate(9), Endosulfan(a-Benzoepin), Endosulfan(b-Benzoepin), Endosulfan(Benzoepin), Endrin, Epichlorohydrin, EPN, Equol, Esprocarb, Estriol, Estrone, Ethanolamine, Ethyl 4-hydroxybenzoate, Ethyl benzene, Ethyl parathion, Ethylcarbamate, Ethylene glycol, Ethylene glycol |
| monoethyl ether, Ethylenediaminetetraacettic acid 2Na, Eugenol, Fenbutatin oxide, Fenitrothion, Fenobcarb, Fenvalerate, Feruic acid, Flavone, Fluazifop-butyl, Flucythrinate, Fluvalinate, Formaldehyde, Genistein, Genistin, g-Hexachlorocyclohexane, Glutaraldehyde, Glycitein, Glycitin, Glycyrrhizic acid 2K, Glyoxal, Heptachlor epoxide, Hexachloro-1,3-butadiene, Hexachlorophene, Hinokitiol, Hinokitiol acetylglucoside, Hinokitiol glucoside, Hippuric acid Na, Hydroquinone, Hydroxy-flutamide, Hydroxylamine sulfate, Hydroxy-tetrachlorobiphenyl, Hydroxy-trichlorobiphenyl, IBP, Iprodione, Isoeugenol, Isophorone, Isoprothiolane, Isoxathion, Kaempferol, Kelthane, Kojic acid, Lead nitrate, Linuron, Malaoxon, Malathion, Maneb, Manzeb, Marthasteroside A1, Mefenacet, Melanine, MelQx, Menadione, Mercury(II) sulfate, Merhyl methacrylate, Metamitron, Methidathion, Methomyl, Methoxychlor, Methyl, 4-hydroxybenzoate, Methylmercury chloride, Metribzin, Microcystin RR, Molinate, Monochloroacetic acid, Monoethoxyleneglycol dimethacrylate, Morpholine, N,N-Dimethylaniline, N,N-Dimethylformamide, Naphtahalene, Naringenin, n-Butyl acrylate, n-Butylbenzene, n-Decyl alcohol, Nebron, Neophethylglycol dimethacrylate, N-Ethylaniline, Nichel(II) chloride, Nitrilotriacetic acid, Nitrofen, n-Methyl 4-hydroxybenzoate, N-Nirtosodiethylamine, N-Nitrosodimethylamine, N-Nitrosodiphenylamine, Nonoxynol iodide, N-Phenyl-1-naphthylamine, N-Phenyl-2-naphthylamine, n-propyl 4-hydroxybenzoate, o,p'-DDD, o,p'-DDE, o,p'-DDT, o-Tolidine, p,p'-DDD, p,p'-DDE, p,p'-DDT, Paraquat, Pendimethalin, Pentachloronitrobenzene, Pentachlorophenol, Permethrin, Phenol, Phenthoate, Phenylhydrazine, Phloretin, PhIP, Phosalone, Polyalkilpolyaminoethylglycine HCl, Potassium cyanide, Pottasium dichromate(IV), Pretiachlor, Primiphos-methyl, Procymidone, Propanil, Propazin, Propoxur, Propyzamide, Pyrene, Quercetin, Quinolin, Resorcinol, Simazine, Simetryne, Sodium arsenite, Sodium lairyl sulfate, Sodium molybdate, Sodium selenate, Styrene, Tamoxifen, Tefluthrin, Terephtalicacid, Teststerone, Tetrabromobisphenol A, Tetrachlorobisphenol A, Tetrachloroethylene, Tetrachlorofthalide, Tetrachlorovinphos, Tetraethylenepentamine, Thallium(I) chloride, Thiabendazole, Thiobencarb, Thiophanate-methyl, Thiourea, Thiram, Thymol, Toluene, Toxaphene, trans-1,2-Diphenylcyclobutane, trans-Stilbene, Triadimefon, Triadimenol, Tributyl phiosphate, Tributyltin(IV) chloride, Triethoxyleneglycol dimethacrylate, |
| Triethylamine, Triethylenetetraamine, Trifluralin, Triforine, Trimethylpropane triacrylate, Trimethylpropane trimethacrylate, Triphenyltin(IV) chloride, Tris(2-chloroethyl) phosphate, Tris(butoxyethyl) phosphate, Trp-P-2, Tyramine, Tyrosine, Urethane dimethacrylate, Vinclozolin, Vinylacetic acid, Xylylcarb, Zineb, Ziram |

## Claims

1. A transformed plant, which is transformed by transducing effector 1 sequence, effector 2 sequence, a target DNA and a reporter gene, wherein said effector 1 sequence has a promoter, a nuclear localization signal, and a chimeric gene comprising a domain coding a polypeptide binding to a target DNA and a domain coding a ligand binding polypeptide of intranuclear receptors for estrogen-like chemicals, said effector 2 sequence has a promoter, a nuclear localization signal, and a chimeric gene comprising a domain coding an interacting domain with intranuclear receptors in a transcriptional coactivator and a domain coding a transcriptional activation domain, and said reporter gene locates in the down stream of said target DNA.

2. A method for detecting an estrogen-like chemical, comprising the steps of contacting said transformed plant of claim 1 with an estrogen-like chemical and detecting the expression of said reporter gene in said plant.

## Patentansprüche

1. Transformierte Pflanze, die durch Transduktion der Effektor 1-Sequenz, der Effektor 2-Sequenz, einer Ziel-DNA und eines Reportergens transformiert wurde,
wobei die Effektor 1-Sequenz einen Promotor, ein Kernlokalisierungssignal und ein dimäres Gen aufweist, das eine Domäne umfasst, die eine Polypeptidbindung zu einer Ziel-DNA codiert und eine Domäne umfasst, die ein Liganden-bindendes Polypeptid eines intranuklearen Rezeptors für östrogenähnliche Substanzen codiert;
die Effektor 2-Sequenz einen Promotor, ein Kernlokalisierungssignal und ein chimäres Gen aufweist, das eine Domäne unfasst, die eine Interaktions-Domäne mit einem intranuklearen Rezeptoren in einem transkriptionellen Co-Aktivators codiert und eine Domäne umfasst, die eine transkriptionelle Aktivierungsdomäne codiert; und
das Reportergen stromabwärts in der Ziel-DNA lokalisiert ist.

2. Verfahren zum Nachweis einer östrogenartigen Substanz, welches die Schritte umfasst: In Kontakt bringen der transformierten Pflanze aus Anspruch 1 mit einer östrogenartigen Substanz und Nachweisen der Expression des Reportergens in der Pflanze.

## Revendications

1. Plante transformée, qui est transformée par transduction d'une séquence effectrice 1, d'une séquence effectrice 2, d'un ADN cible et d'un gène rapporteur, dans laquelle ladite séquence effectrice 1 a un promoteur, un signal de localisation nucléaire, et un gène chimérique comprenant un domaine codant pour un polypeptide se liant à un ADN cible et un domaine codant pour un polypeptide de liaison aux ligands de récepteurs intranucléaires pour des substances chimiques de type estrogène, ladite séquence effectrice 2 a un promoteur, un signal de localisation nucléaire, et un gène chimérique comprenant un domaine codant pour un domaine d'interaction avec des récepteurs intranucléaires dans un coactivateur transcriptionnel et un domaine codant pour un domaine d'activation transcriptionnelle, et ledit gène rapporteur est situé en aval dudit ADN cible.

2. Procédé de détection d'une substance chimique de type oestrogène, comprenant les étapes de mise en contact de ladite plante transformée selon la revendication 1 avec une substance chimique de type oestrogène et de détection de l'expression dudit gène rapporteur dans ladite plante.
